(19) **Europäisches Patentamt / European Patent Office / Office européen des brevets**

(11) **EP 2 434 997 B1**

(12) **EUROPEAN PATENT SPECIFICATION**

(45) Date of publication and mention
of the grant of the patent:
**29.10.2014 Bulletin 2014/44**

(21) Application number: **10780967.5**

(22) Date of filing: **19.04.2010**

(51) Int Cl.:
***A61F 9/007*** *(2006.01)*

(86) International application number:
**PCT/US2010/031598**

(87) International publication number:
**WO 2010/138258 (02.12.2010 Gazette 2010/48)**

(54) **NON-LINEAR CUT-RATE MULTIPLIER FOR VITREOUS CUTTER**

NICHTLINEARER SCHNEIDRATENVERVIELFACHER FÜR GLASSCHNEIDER

MULTIPLICATEUR DE VITESSE DE COUPE NON LINÉAIRE POUR DISPOSITIF DE COUPE VITREUX

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **03.03.2010 US 717035**
**26.05.2009 US 181199 P**

(43) Date of publication of application:
**04.04.2012 Bulletin 2012/14**

(73) Proprietor: **Medical Instrument Development Laboratories, Inc.**
**San Leandro, CA 94577 (US)**

(72) Inventors:
• **PETERSON, Erik William**
**Walnut Creek, CA 94595 (US)**

• **CHEN, David E-bin**
**Fremont, CA 94539 (US)**

(74) Representative: **DREISS Patentanwälte PartG mbB**
**Patentanwälte**
**Gerokstrasse 1**
**70188 Stuttgart (DE)**

(56) References cited:
**WO-A1-00/78371        JP-A- 2002 125 995**
**US-A1- 2002 173 814        US-A1- 2007 167 943**
**US-A1- 2007 167 943        US-A1- 2008 114 387**
**US-B1- 6 575 990        US-B2- 7 335 217**

**Description**

BACKGROUND OF THE INVENTION

The present invention relates to devices for performing micro-surgical procedures in the posterior portion of the eye. More particularly, the present invention relates to a cut-rate controller for a vitreous cutter.

The instrument most commonly used, and generally preferred, for vitreous surgery is a pneumatically-operated axial guillotine cutter. A typical pneumatically-operated guillotine cutter system includes a handpiece (sometimes called a "cutter") that includes a needle with a cutting/aspiration port located near the needle's distal end. The handpiece receives pneumatic power from a vitreoretinal surgical system (sometimes called a "drive unit" or a "console"). Often, the system also provides aspiration and illumination functions.

Although numerous improvements have been made over the years, the fundamental aspects of vitreous cutters are known and taught by O'Malley and Heintz in U.S. Patent Nos. 3,884,238 and 3,815,604, respectively. In its modern form, the axial guillotine cutter is relatively small, lightweight, durable, inexpensive, and exhibits good cutting characteristics.

One improvement made in vitreous cutting has been the ability to operate at higher cut-rates (the number of cuts made per minute). This generally results in better-controlled and safer cutting. Operation at high cut-rates requires improvements both to the vitreous cutter and to the drive unit. U.S. Patent No. 6,575,990, issued to Wang et al., discloses a means of adding a separate drive unit as an accessory to an existing surgical system (referred to as the "host system") in order to provide for higher cut-rates without modification of the host system. Examples of accessory drive units which embody this patent (referred to as the "accessory drive unit") include the AVE and the VIT Enhancer units (see, http://www.midlabs.com/ave.htm) sold by Medical Instrument Development Laboratories, Inc. of San Leandro, California. An accessory drive unit may include electronics and pneumatic components, such as tubing and a pneumatic valve controlled by the electronics. Some accessory drive units may include internal air compressors.

BRIEF SUMMARY OF THE INVENTION

In available accessory drive units, the cut-rate of the guillotine cutter is set using controls on a front panel. There are some deficiencies in setting the cut-rate in this manner. First, the controls add complexity and cost to the accessory drive unit. Second, because the controls are used to set the cut-rate, the accessory drive unit must be designed so that the controls can be readily accessed by the user of the cutter (e.g., a surgeon). Lastly, the cut-rate can not be varied using the controls of the host surgical system, although in some instances it is possible to provide means for the surgeon to vary the cut-rate using a foot pedal control.

Accordingly, in one embodiment, the invention as claimed in claims 1 and 14 provides an accessory drive unit, in the form of a non-linear cut-rate multiplier, so that the cutter can be driven at a frequency or rate that is different than the rate available from the host surgical system. In this embodiment, the cut-rate varies as a non-linear function of the cut-rate set on the host surgical system.

In another embodiment, the invention provides a non-linear cut-rate multiplier that includes an input sensor that senses an input signal with a first frequency provided by a drive unit, and a non-linear frequency multiplier circuit that receives the input signal and outputs an output signal at a second frequency that is a non-linear multiple of the first frequency. The non-linear cut-rate multiplier also includes a trigger circuit that receives the input signal and outputs a trigger signal. A drive circuit receives the output signal and the trigger signal and outputs an actuation signal with a third frequency. The third frequency is substantially equal to the second frequency.

In another embodiment, the invention as claimed in claim 7 provides a method of controlling the cut-rate of a vitreous cutter. The method includes detecting a drive signal produced by a drive unit. The drive signal drives the vitreous cutter at a first frequency. The method also includes sensing the drive signal with an input sensor of a non-linear cut-rate multiplier; processing the drive signal into an output signal with a second frequency that is a non-linear multiple of the first frequency; and producing an actuation signal to drive the vitreous cutter at a third frequency that is substantially equal to the second frequency.

Other aspects of the invention will become apparent by consideration of the detailed description and accompanying drawings.

BRIEF DESCRIPTION OF THE SEVERAL VIEWS OF THE DRAWING

Fig. 1 is a schematic view of a high-speed vitreous cutting system that includes a host system, a non-linear cut-rate multiplier, and a vitreous cutter;

Fig. 2 is a schematic diagram of one embodiment of a non-linear cut-rate multiplier circuit suitable for use in the present invention;

Fig. 3 is a circuit diagram illustrating additional detail of a non-linear frequency multiplier circuit, which is part of the cut-rate multiplier circuit shown in Fig. 2;

Fig. 4 is a schematic diagram of a second embodiment of a non-linear cut-rate multiplier circuit suitable for use in the present invention;

Fig. 5 is a circuit diagram illustrating additional detail of a track and hold circuit, which is part of the cut-rate multiplier circuit shown in Fig. 2;

Fig. 6 is a schematic diagram of one embodiment of a non-linear cut-rate multiplier microcontroller unit suitable for use in the present invention;

Fig. 7 is an example of a host system selection table used in the vitreous cutting system of Fig. 1;

Fig.8 is an alternative example of a host system selection table used in the vitreous cutting system of Fig. 1;

Fig. 9 is a graph illustrating several functions for adjusting the cut-rate frequency of the vitreous cutting system using the non-linear cut-rate multiplier of Fig. 1;

Fig. 10 is a flow chart of a method for operating the non-linear cut-rate multiplier of Fig. 1; and

Fig. 11 is a perspective view of a switch mechanism used with the high-speed vitreous cutting system of Fig. 1.

DETAILED DESCRIPTION OF THE INVENTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

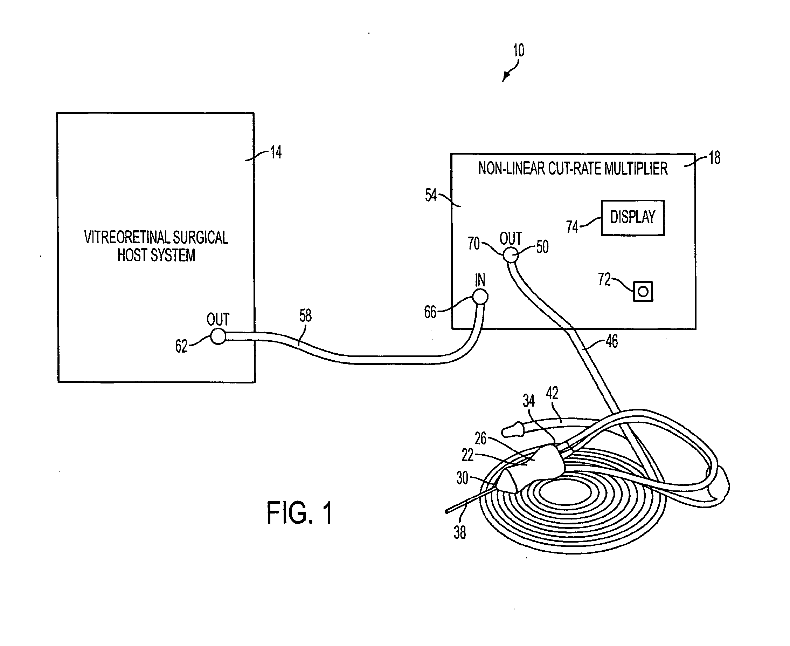

Fig. 1 illustrates a high-speed vitreous cutting system 10 that includes a vitreoretinal surgical host system 14 (herein referred to as a "drive unit"), an accessory in the form of a non-linear cut-rate multiplier 18, and a pneumatically-operated axial guillotine cutter 22 (herein referred to as a "cutter"). The drive unit 14 may be any unit typically used in vitreoretinal surgery that is operable to output a signal to drive a pneumatically or electrically actuated cutter.

The cutter 22 is a pneumatically driven, axial guillotine-type vitreous probe or cutter. One such cutter is described in detail in U.S. Patent No. 6,575,990, filed October 20, 2000. The illustrated cutter 22 contains a generally cylindrically shaped housing 26 designed to be held in a human hand. The housing has a first end 30 and a second end 34. A needle 38 is coupled to the first end 30. The needle 38 includes a cutting or aspiration port near the distal end for use in removing vitreous.

Tubing, having two adjacent tubes 42 and 46, is connected to the cutter 22. One end of the aspiration tube 42 is connected to a port on the second end 34 of the cutter 22. Actuation tube 46 is also coupled to the second end 34 of the cutter 22. The other end of the actuation tube 46 is connected to a fitting 50, such as a male, luer-lock fitting, located on a front panel 54 of the non-linear cut-rate multiplier 18. In addition to the pneumatically driven cutter described below, in other embodiments, the cutter may be an electrically actuated cutter. (The electric signal could be provided to an electrically-controlled valve to control pneumatic pulses or to a solenoid that produces linear motion to drive an electrically-controlled cutter).

The non-linear cut-rate multiplier 18 is illustrated as a separately housed accessory in the vitreous cutting system 10. A tubing 58 is connected to an output port 62 on the drive unit 14 and to an input port 66 on the non-linear cut-rate multiplier 18. The non-linear cut-rate multiplier 18 also includes an output port 70. The fitting 50 is connected to the output port 70. The non-linear cut-rate multiplier 18 may include a pneumatic drive system (e.g., an electric air compressor) for providing pneumatic energy or compressed air to the cutter 22. A back panel of the non-linear cut-rate multiplier 18 includes a socket for connecting an electrical cord with a plug for supplying electric power to the transformer and a power switch for turning the non-linear cut-rate multiplier 18 on and off.

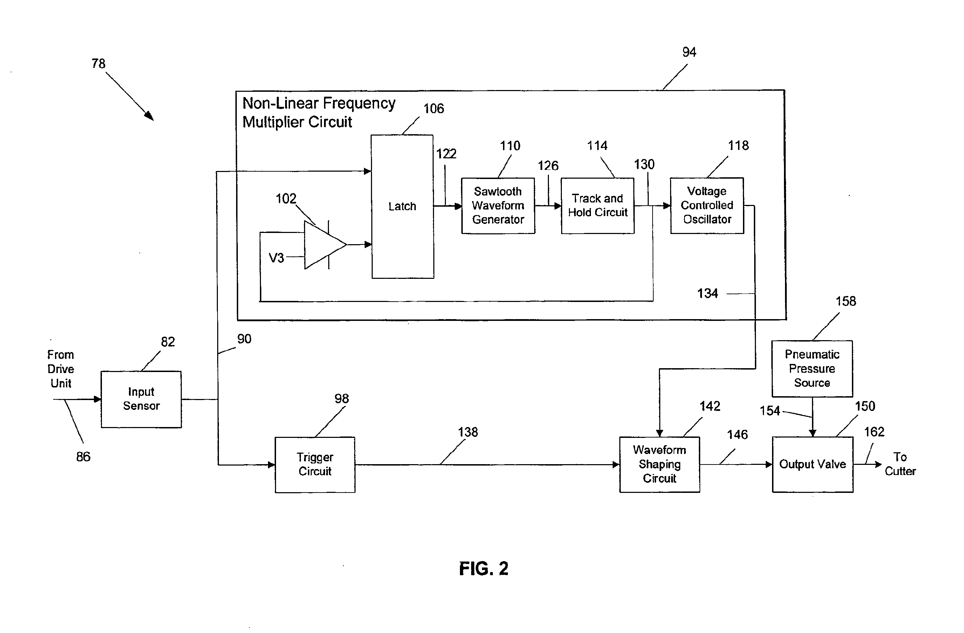

In some drive units, the available frequencies may range from approximately 400 cuts/min to 750 cuts/min. In other drive units, the available frequencies may range from 400 cuts/min to 2,500 cuts/min. With the use of the non-linear cut-rate multiplier 18, the range of available frequencies increases. For example, the available frequencies can range from 400 cuts/min to 12,000 cuts/min. The range of frequencies available from the non-linear cut-rate multiplier 18 is determined by a non-linear function (illustrated as Equation 1 and described below), which is determined by the circuit parameters of the non-linear frequency multiplier circuit 94 (Fig. 2). Thus, the user can achieve cut-rates that are significantly greater than the cut-rates available from the drive unit 14 alone. In alternative embodiments, the range of frequencies from the multiplier 18 also can be determined by a linear function. This is often desirable when performing vitreoretinal surgery because lower cutting rates are used for rapid removal of vitreous in the center part of the eye while higher cutting rates are provided for more controlled removal of vitreous near the retina. In other constructions, the non-linear cut-rate multiplier 18 may provide options for adjusting the function or choosing a predefined function using a switch or other selection mechanism. Alternately, a two or more position rotating dial may be rotated by the user to choose one of two or more different functions for adjusting the frequency. Thus, two drive units, with two different ranges of available frequencies (e.g., 400 cuts/min to 750 cuts/min and 400 cuts/min to 2,500 cuts/min) can be used with the non-linear cut-rate multiplier 18 to each produce the same range of frequencies (e.g., 400 cuts/min to 12,000 cuts/min) by choosing an appropriate function.

In operation, the non-linear cut-rate multiplier 18 receives a signal from the drive unit 14 through tubing 58, which is connected to the input port 66. The front portion of the non-linear cut-rate multiplier 18 includes a front panel button 72 and a display 74. A user can select the type of drive unit 14 (host system) connected to the non-linear cut-rate multiplier 18 from a variety of drive/host systems shown on the display 74 by pressing the button 72. The display 74 can show the available host systems, along with their corresponding cut rates and cut rate control forms (footpedal, panel, or other). As indicated above, the front panel button 72 also can be used to adjust or select a specific function. The back panel of the non-linear cut-rate multiplier 18 can include a rear power switch (not shown) that activates the front panel button 72. The non-linear cut-rate multiplier 18 processes the signal and outputs an actuation signal that drives the cutter 22. The frequency of the actuation signal is a non-linear multiple of the frequency of the signal from the drive unit 14 such that the cutter 22 may be operated at a faster speed or rate than possible using the drive unit 14 alone. In addition, the non-linear cut-rate multiplier 18 allows switchability between a non-linear cut-rate and a linear cut-rate, and the multiplied signal may be outputted at both non-linear and linear cut-rates to the vitreous cutter.

To illustrate the advantages and/or improvements of non-linear multiplication over linear multiplication in operating the high-speed vitreous cutting system 10, the following example should be considered. The host system 14 in this example has a practical control range of 150 cuts/min to 800 cuts/min. The accessory device (multiplier 18) can be used to give cut rates up to 8000 cuts/min, but is mostly used in the 2000 to 4000 cuts/min range. In addition, there are certain situations where it might be desirable for a user to have a cut rate as low as 300 cuts/min. At the upper limit of the range (800 cuts/min input, 8000 cuts/min output) a multiplication factor of 10 is required. At the lower limit of the range (150 cuts/min input, 300 cuts/min output) a multiplication factor of 2 is required. With linear (fixed) multiplication, it would be necessary to stop at various times during the surgical procedure to select different multiplication factors. In contrast, the non-linear cut-rate multiplier 18 can easily be set up to have the required multiplication factors of 2 at 150 cuts/minute input and 10 at 800 cuts/minute input. Moreover, the non-linear cut-rate multiplier 18 would have an effective multiplication factor of approximately 4.8 at the center of the most-commonly-used range.

Fig. 2 illustrates one embodiment of a non-linear cut-rate multiplier circuit 78 (shown in schematic form) that may be used by the non-linear cut-rate multiplier 18. An input sensor 82 senses the (pneumatic or electrical) signal 86 from the drive unit 14 and outputs an alternating current (AC) signal 90 with the same frequency to the non-linear frequency multiplier circuit 94 and to a trigger circuit 98.

The cut-rate multiplier circuit 78 includes a non-linear frequency multiplier circuit 94. The circuit 94 includes a comparator 102, a latch 106, a sawtooth waveform generator 110, a track and hold circuit 114, and a voltage controlled oscillator ("VCO") 118. The latch 106 receives the AC signal 90 and a signal from the comparator 102. The latch 106 produces a square-wave output 122 in response to the received signals. The sawtooth waveform generator 110 receives the square-wave signal 122 and generates a sawtooth waveform 126. The track and hold circuit 114 receives the sawtooth waveform 126 and tracks a minimum value of the sawtooth waveform 126. The track and hold circuit 114 produces a direct current (DC) output 130, which is provided to the VCO 118. The VCO 118 produces an AC output 134 with a frequency that varies under the influence of (or is based upon) the signal 130 received from the track and hold circuit 114. (The AC output 134 is also the output of the circuit 94.)

The trigger circuit 98 generates a DC signal 138 which is provided to a waveform shaping circuit 142. The DC signal 138 is a logical signal that is a high voltage, typically about 5 volts, when the signal 90 is detected by the trigger circuit 98 and a low voltage, typically about 0 volts, when the signal 90 is not detected by the trigger circuit 98. The DC signal 138 enables the waveform shaping circuit 142 when it is a high voltage and disables the waveform shaping circuit 142 when it is a low voltage. The waveform circuit 142 also receives the AC output 134 of the VCO 118. One example of a waveform shaping circuit is described in U.S. Patent No. 6,575,990, filed on October 20, 2000. The waveform shaping circuit 142 processes the signals 134 and 138 to produce a square-wave, output signal 146. The output signal 146 is provided to a solenoid output valve 150. The solenoid output valve 150 is a three-way valve and receives pressurized gas 154 (or pressurized air) from a pressurized gas source 158. The signal 146 causes the solenoid valve 150 to open and close, which results in the generation of a gas pulse train (or pneumatic signal) 162. The gas pulse train is provided to the cutter 22 to pneumatically operate or drive the cutter 22 at a frequency that approximates the frequency of the output signal 134 of the non-linear frequency multiplier circuit 94. The solenoid output valve 150 is vented when not actuated.

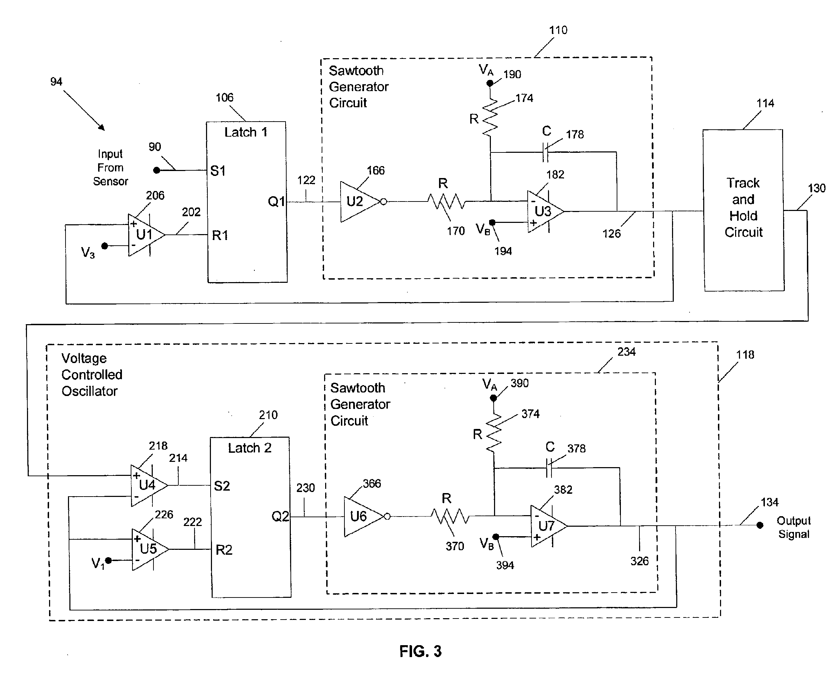

Fig. 3 illustrates the non-linear frequency multiplier circuit 94 in more detail. Although one particular example is illustrated and explained herein, other circuits can be designed that perform similar functions. The non-linear frequency multiplier circuit 94 receives the AC input signal 90 from the input sensor 82. The signal 90 has a frequency F1. The output signal 134 has a frequency F2. Equation 1 is a non-linear function that describes the relationship between the frequencies F1 and F2, where $\alpha$ and $\beta$ are positive constants determined by the circuit parameters of the non-linear frequency multiplier circuit 94, as described below.

$$F2 = F1\left(\frac{1}{\alpha - \beta\, F1}\right) \qquad \qquad \text{Equation 1}$$

The non-linear frequency multiplier circuit 94 is designed such that when the frequency F1 of the input signal 90 is low, the frequency F2 of the output signal 134 is approximately equal to the frequency F1 of the input signal 90. As the frequency F1 of the input signal 90 increases, the frequency F2 of the output signal 134 increases at a faster, non-linear rate that is determined by the circuit parameters such as resistance, capacitance, input bias voltage, etc. Such a relationship has certain benefits for a surgeon performing vitreoretinal surgery. For example, when a surgeon is operating a cutter at a low frequency (i.e., a low cut rate), which is commonly used for rapidly removing vitreous, the surgeon can make more controlled adjustments to the output frequency because the output frequency is nearly equal to the input frequency at low cut rates. When the surgeon is operating at a high frequency, less vitreous is removed over time. In general, the higher the frequency, the slower the removal of vitreous. Thus, the non-linear frequency multiplier circuit 94 allows the surgeon to operate at higher frequencies (i.e., higher cut-rates) than achievable from the drive unit 14 alone, thereby providing the surgeon with better control over the removal of vitreous.

The specific aspects of the circuit 94 that help achieve the advantages noted above include the sawtooth generator circuit 110. As shown in Fig. 3, the sawtooth waveform generator circuit 110 includes an open-collector driver 166, a first resistor 170, a second resistor 174, a capacitor 178, and an operational amplifier 182. The open-collector driver 166 (alternatively an open-drain driver could be used) acts similar to a switch to short its output 126 to ground when activated and to allow its output 126 to float when not activated. When the open-collector driver 166 is not activated, the output 126 of the operational amplifier 182 integrates downward at a rate which is determined by the values R of the resistors 170 and 174, the value C of the capacitor 178, and the values $V_A$ and $V_B$ of the voltages at nodes 190 and 194, respectively. When the open-collector driver 166 is activated, the output 126 of the operational amplifier 182 integrates upward at a rate determined by the values R of the resistors 170 and 174, the value C of the capacitor 178, and the values $V_A$ and $V_B$ of the voltages at nodes 190 and 194, respectively.

The latch 106 receives the input signal 90 from the input sensor 82. The latch 106 is set when the input signal 90 rises above a first threshold, which is determined by the latch and is typically about 5 volts. When the latch 106 is set, the output 122 of the latch 106 is high and the open-collector driver 166 is activated. The latch 106 also receives a signal 202 from a comparator 206. When the output signal 126 of the sawtooth waveform generator circuit 110 rises above a voltage $V_3$, the comparator 206 outputs a high voltage, typically about 5 volts, to reset the latch 106. When the latch 106 is reset, it outputs a low voltage, typically about 0 volts, which is received by the open-collector driver 166. When the open-collector driver 166 receives a low voltage, the open-collector driver 166 is not activated and the output of the open-collector driver 166 floats. Thus, the output 126 of the sawtooth waveform generator circuit 110 begins to integrate downward at a fixed rate, until the latch 106 is again set by the input signal 90. When the input signal 90 to the latch 106 is a signal such as a periodic pulse train with a frequency F1, the output 126 of the sawtooth waveform generator circuit 110 is a sawtooth waveform with approximately the same frequency as the input signal 90. The sawtooth waveform oscillates between a maximum voltage equal to $V_3$ and a minimum voltage arbitrarily denoted as $V_x$. The minimum voltage $V_x$ depends on the frequency of the input signal 90 and the rates at which the signal 126 integrates upward and downward (i.e., the slopes of the rise and fall of the sawtooth waveform).

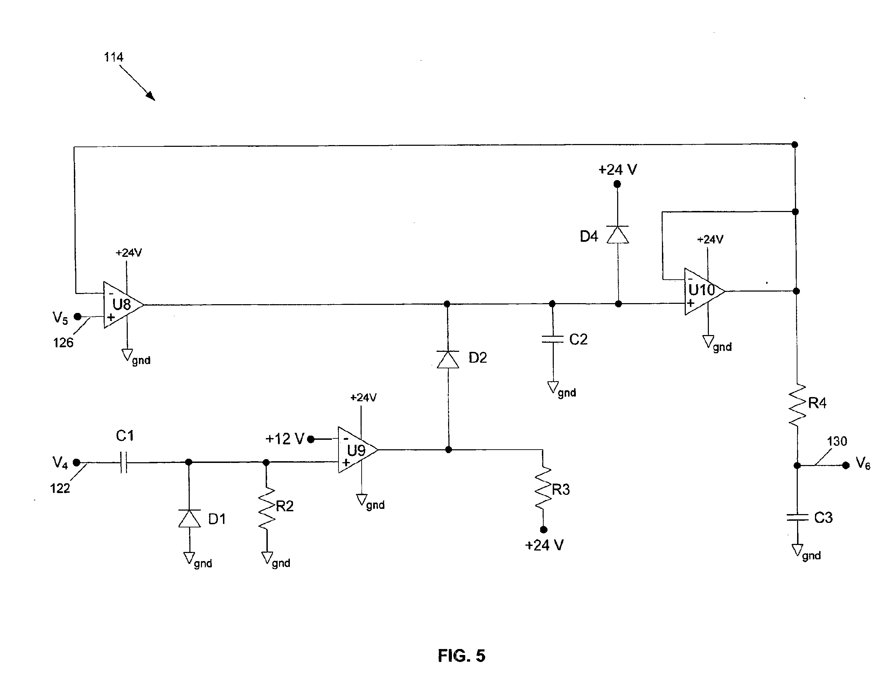

The track and hold circuit 114 receives the output 126 from the sawtooth waveform generator circuit 110 and outputs a DC voltage 130 that tracks with the minimum voltage $V_x$ of the output 126 of the sawtooth generator circuit 110. One example of a track and hold circuit is illustrated in Fig. 5, where the track and hold circuit 114 receives the output 126 from the sawtooth waveform generator circuit 110 as an input signal $V_5$ and receives the square wave output 122 from the latch 106 as an input signal $V_4$. The track and hold circuit 114 outputs the DC voltage 130 as an output signal $V_6$. The minimum voltage $V_x$ of the output 126 of the sawtooth generator circuit 110 can be determined from Equation 2, where A is a constant determined by the component and bias voltage values in the sawtooth waveform generator circuit 110.

$$V_x = V_3 - A\frac{1}{F1} \qquad \text{Equation 2}$$

The square wave signal 122 transitions from a low voltage to a high voltage when the signal 126 is at the minimum voltage $V_x$. The track and hold circuit 114 samples the voltage of signal 126 on the low-to-high transition of the square wave signal 122. Thus, the output 130 of the track and hold circuit 114 is approximately equal to the minimum voltage $V_x$ of signal 126. The voltage controlled oscillator 118 receives the minimum voltage $V_x$ and produces an output signal 134 with a frequency F2 that can be determined according to Equation 3, where B is a constant determined by the circuit parameters of the VCO 118.

$$\frac{1}{F2} = B(V_1 - V_x) \qquad \text{Equation 3}$$

More specifically, the VCO 118 includes two comparators 218 and 226, a latch 210, and a sawtooth generator circuit 234. The comparator 218 outputs a high voltage when the output 134 of the sawtooth waveform generator circuit 234 is below the voltage of the output signal 130 of the track and hold circuit 114. The comparator 226 outputs a high voltage when the output 134 of the sawtooth waveform generator circuit 234 is above $V_1$. The latch 210 receives the signal 214 output by comparator 218 and the signal 222 output by comparator 226. The latch 210 is set and outputs a high voltage 230 when the signal 214 from comparator 218 is high (typically about 5 volts). The latch 210 is reset and outputs a low voltage 230 when the signal 222 from comparator 226 is high (typically about 5 volts).

The sawtooth waveform generator circuit 234 is similar to the sawtooth waveform generator circuit 110 and operates in a similar manner. As illustrated, the circuit components have the same nominal values (e.g., R and C), are connected in a similar manner, and perform similar functions. Like components have been given like reference numbers of the 300 series. When the open-collector driver 366 receives a high input voltage 230, the output 134 of the sawtooth waveform generator circuit 234 integrates upward, or increases linearly. When the open-collector driver 366 receives a low input voltage 230, the output 134 of the sawtooth waveform generator circuit 234 integrates downward, or decreases linearly.

Thus, the output 134 of the sawtooth waveform generator circuit 234 increases until the output 134 becomes greater than $V_1$, at which point, the output of the comparator 226 becomes high and resets the latch 210. Then, the output 134 of the sawtooth waveform generator circuit 234 begins to decrease until the output 134 becomes less than the output 130 of the track and hold circuit 114, at which point the comparator 218 outputs a high voltage to set the latch 210. When the latch 210 is set, the output 134 begins to increase again. The cycle repeats itself such that the output of the non-linear frequency multiplier circuit 94 outputs a sawtooth waveform that oscillates between $V_x$ and $V_1$ with a frequency F2 described by Equation 3.

Equation 3 can be simplified by substituting Equation 2 into Equation 3 and simplifying. After simplification, Equation 3 can be rewritten as follows.

$$F2 = F1\left(\frac{1}{AB - B(V_3 - V_1)F1}\right) \qquad \text{Equation 4}$$

As can be seen by comparing Equation 1 to Equation 4, $\alpha$ is equal to AB and $\beta$ is equal to $B(V_3-V_1)$. Equations 1 and 4 may be used interchangeably. For simplicity, Equation 1 is disclosed to the user of the cut-rate multiplier 18. In some constructions, the cut-rate multiplier 18 may include user options that allow the user to select the desired value of $\beta$, perhaps by adjusting a switch on the front panel 54 of the non-linear cut-rate multiplier 18 or otherwise selecting values for $\beta$. For example, a switch may be provided on the front panel 54 to allow the user to select a voltage for $V_3$,

which is applied to the negative input of the comparator 206. Alternatively, a switch may be provided on the front panel 54 to allow the user to select a voltage for $V_1$, which is applied to the negative input of the comparator 226.

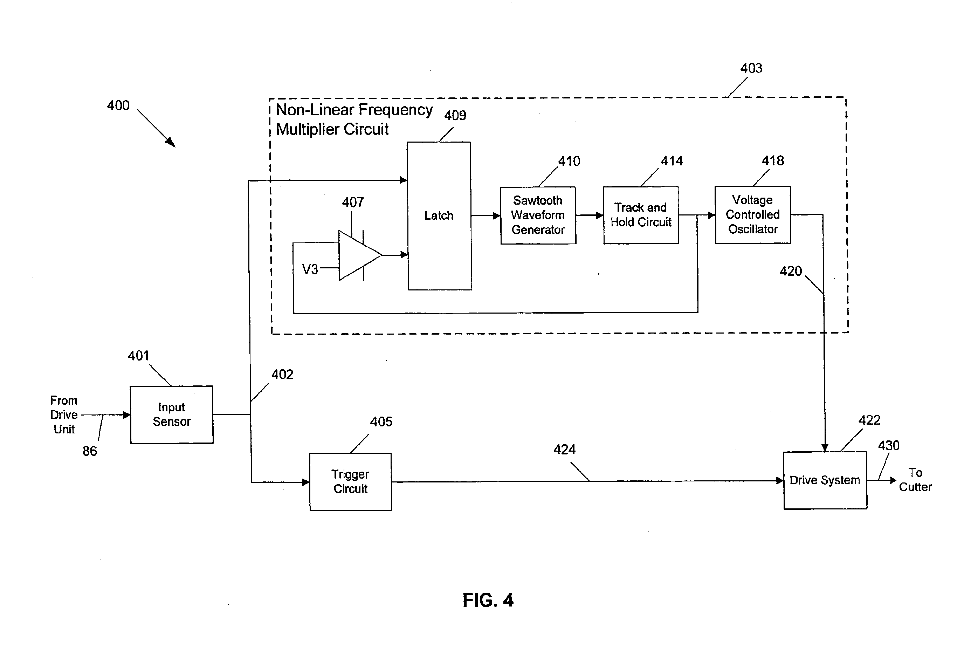

Fig. 4 illustrates a second embodiment of a non-linear cut-rate multiplier circuit 400 (shown in schematic form). An input sensor 401 senses the signal 86 from the drive unit 14 and outputs an AC signal 402 with the same frequency to a non-linear frequency multiplier circuit 403 and to a trigger circuit 405.

The multiplier circuit 403, which is similar to the non-linear frequency multiplier circuit 94 of Fig. 2, includes a comparator 407, a latch 409, a sawtooth waveform generator 410, a track and hold circuit 414, and a VCO 418, which all operate in a similar way as described with respect to Fig. 2. The VCO 418 generates an output signal 420, which is an AC signal with a frequency that is a non-linear multiple of the frequency of the input signal 86. A drive system 422 receives an output signal 424 from the trigger circuit 405 and the AC signal 420. The drive system 422 converts the signals received into an electrical signal 430 that drives an electrically actuated vitreous cutter.

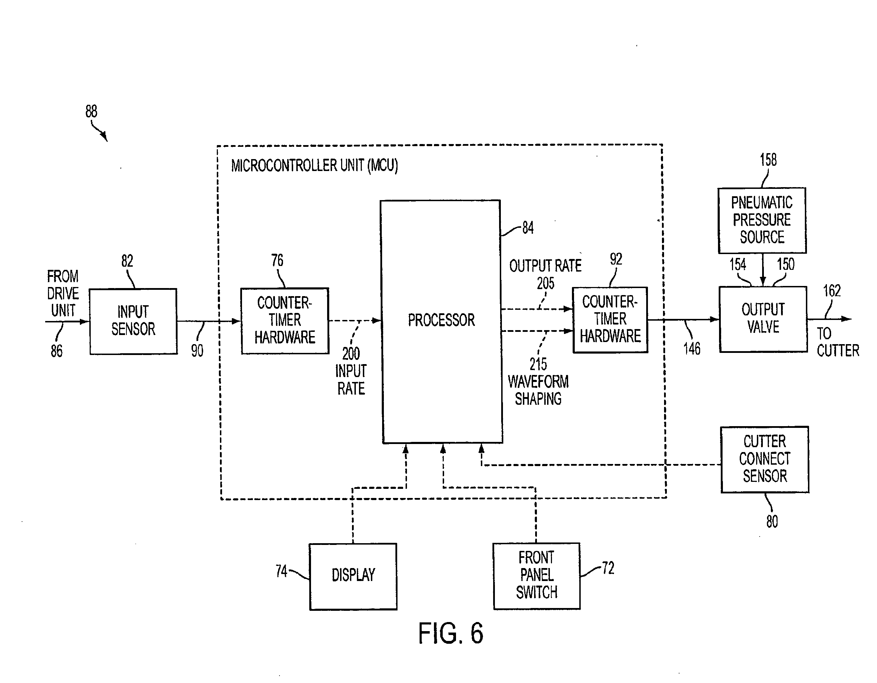

In an alternative embodiment, instead of using the analog frequency multiplier circuit 78, the variable non-linear cut-rate multiplier 18 is driven or operated with digital components by a microcontroller unit ("MCU") 88. As shown in Fig. 6, the microcontroller unit 88 includes an input counter-timer hardware 76, a processor 84, and an output counter-timer hardware 92, where all components are located on a single chip. In other embodiments, the MCU 88 can include additional components that are not located on a single chip. An input sensor 82 senses the signal 86 from the drive unit 14 and outputs an alternating current ("AC") signal 90 with the same frequency to the MCU 88.

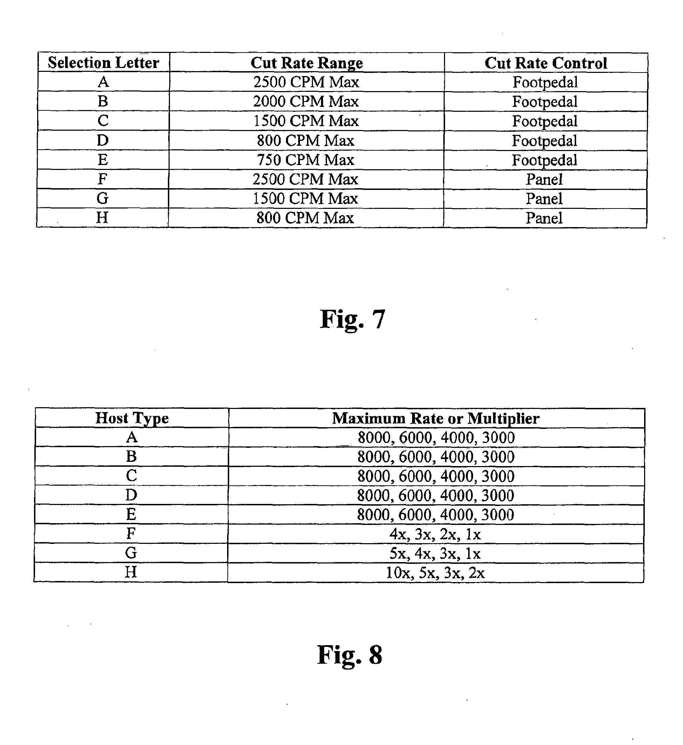

In addition, the processor 84 is connected to the display 74, which displays various types of hosts systems (drive units 14) and corresponding cut rates that can be selected by a user via the front panel button 72. Fig. 7 shows an example of a host system selection table that is available to a user via the display 74 and the front panel button 72. Additional data or elements can be included in the table, as will be apparent to those skilled in the art. Further, the processor 84 of the MCU 88 is connected to a cutter connect sensor 80 that can transmit various conditions of the cutter 22. Using the information received from the cutter connect sensor 80, the processor 84 can determine whether the cutter 22 is working properly by executing a cutter test, which is described in more detail below.

During the cutter test, the processor 84 receives signals from the cutter connect sensor 80 and uses various calculations to determine whether the cutter 22 is defective in any way and thus can be dangerous to the patient. For example, by measuring the pressure and the volume of circulating gas, the cutter test looks for leaks in tubes 42 and 46 that lead to a potential malfunction of the cutter 22. In addition, the cutter test can be used to determine if any other or all components of the cutter 22 function properly.

Conventional methods for initiating the cutter test require that the operator of the cutter 22 performs an action involving controls/switches in order to start the test (e.g., press a button). In an embodiment of the invention, the cutter test is initiated automatically from the act of connecting the cutter 22 to the multiplier 18. This automatic initiation of the cutter test helps to avoid a potential mistake or neglect by a human operator and verifies that there are no hazards associated with the cutter 22.

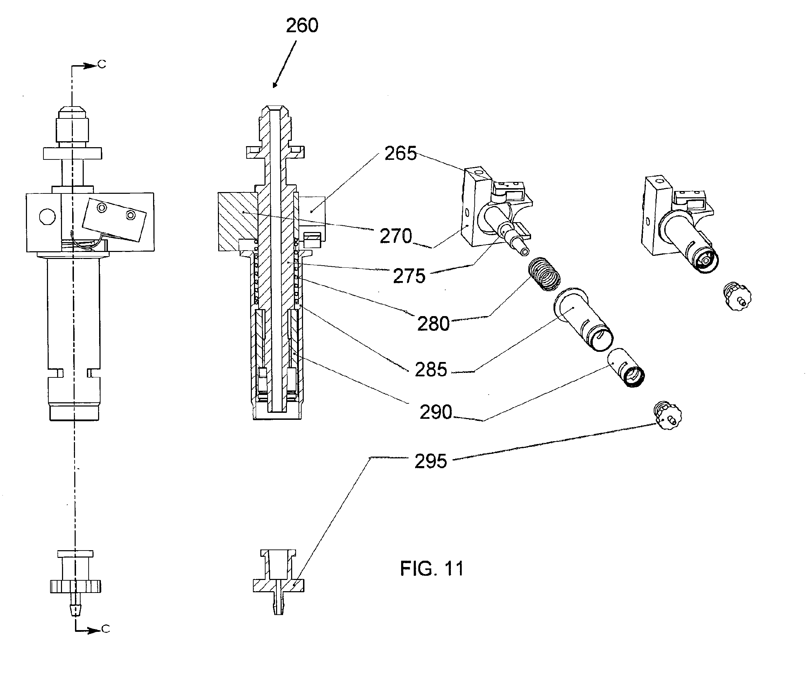

The cutter test is initiated by a mechanical switch 265 coupled to a switch mechanism 260 (Fig. 11), where the switch 265 is automatically "pressed" based on the motion of connecting the cutter 22 to the multiplier 18. As shown in Fig. 11, the switch mechanism 260 includes the switch 265, a switch block 270 that engages and supports the switch 265 in a position relatively adjacent to the switch block 270 and in a position connected to a luer shaft 275, which pneumatically links a vitrectomy probe luer connector 295 to the multiplier 18. The switch mechanism 260 further includes a switch actuator 285 that slides inwardly on the top surface of the luer shaft 275 and includes a flange (not shown) that actuates the switch 265, a spring 280 that releases the switch actuator 285 from the switch 265 when the probe luer connector 295 is not connected to the multiplier 18, and a luer lock 290 that engages with the luer shaft 275 to secure the switch actuator 285 and the spring 280 on the luer shaft 275. The vitrectomy probe luer connector 295 is positioned into the luer lock 290 and includes an inwardly opening that engages the luer head of the luer shaft 275, where the probe luer connector 295 provides pressure to "push" the switch actuator 285 to activate the switch 265 when the luer connector 295 is connected to the multiplier 18. Additional embodiments and elements of the switch mechanism 260 can also be used and will become apparent to those skilled in the art. In an embodiment, the cutter test is performed by the processor 84 of the microcontroller unit 88 and the switch mechanism 260 is located within the body of the multiplier 18. In an alternative embodiment the multiplier 18 and the drive unit 14 can be consolidated into a single unit that is connected to and controls the cut-rate of the cutter 22. In that situation, the cutter test is performed by the controller of the consolidated unit and the switch mechanism 260 is located within the body of the consolidated unit.

In an embodiment of the invention, the input counter-timer hardware 76 receives an initial AC signal 90 sent from the input sensor 82 and measures the period ($P_{in}$) between pulses received from the input sensor 82 in order to determine the frequency of the received signal. The counter-timer hardware 76 then transmits a signal 200 to the processor 84 that modifies the frequency of the signal 200 using non-linear multiplication. The modification (multiplication) of the frequency in the processor 84 is based on different functions for adjusting frequency that are inputted by a user via the front panel button 72. Any host system (drive unit 14) can have a predetermined cut rate range as shown in Fig. 7. In addition, during a surgical procedure a user can adjust the cut rate by changing the Output Maximum Cut Rate or the Cut Rate Multiplier of the host device (drive unit 14). Examples of different Output Maximum Cut Rates and Cut Rate Multipliers are shown in Fig. 8 but other variations will be apparent to those skilled in the art.

After the processor 84 multiplies the received cut-rate signal, the processor 84 transmits the modified frequency in the form of two signals/commands to the output counter-timer hardware 92. The 205 signal/command determines the output period ($P_{out}$) by which the counter-timer hardware 92 controls the timing between electrical pulses (signals) transmitted to the cutter 22. The output period $P_{out}$ is based on the non-linear frequency calculations performed in the processor 84. The 215 signal/command determines the waveform shaping of the signal transmitted to the cutter 22 via output counter-timer hardware 92 in the same way as previously described with respect to Fig. 2.

The output counter-timer hardware 92 produces an output signal 146 based on the signals received from the processor 84. The output signal 146 is provided to a solenoid output valve 150. The solenoid output valve 150 receives pressurized gas 154 (or pressurized air) from a pressurized gas source 158. The signal 146 causes the solenoid valve 150 to open and close, which results in the generation of a gas pulse train (or pneumatic signal) 162. The gas pulse train is provided to the cutter 22 to pneumatically operate or drive the cutter 22 at a frequency that approximates the frequency of the output signal 162 of the microcontroller unit 88.

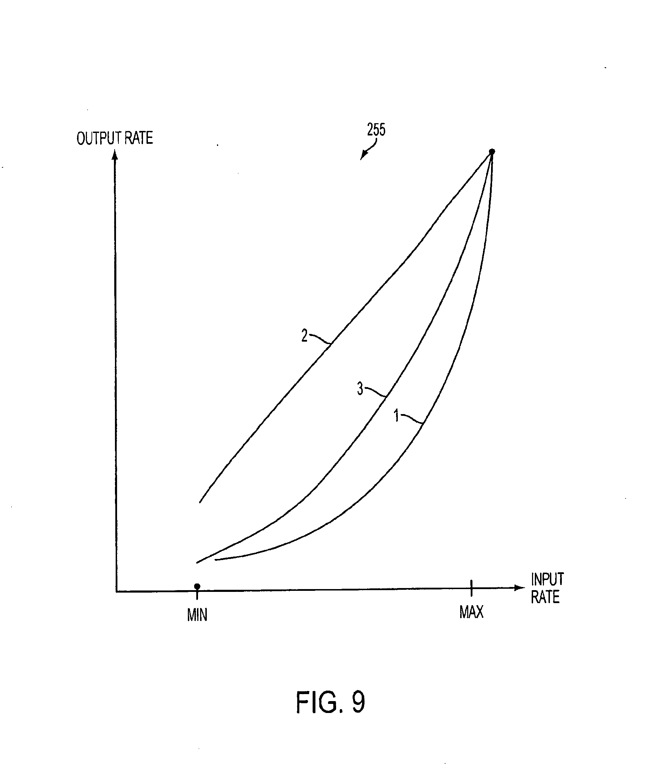

Fig. 9 shows a graph 255 that represents several functions for adjusting cut-rate frequency that can be executed by the processor 84 of the microcontroller unit 88. The front panel button 72, or any other type of user control, can be used to configure the processor 84 to execute these functions for adjusting frequency (transformation functions). In Fig. 9, the x-axis of the graph 255 represents the input rate of the signal from the drive unit 14 and the y-axis represents the output rate of the signal after the non-linear multiplication performed by the processor 84. Function (1) of the graph represents the equation $P_{out} = A*P_{in} - B$ (where A and B are constants) that is mathematically equivalent to the non-linear frequency multiplication of the input signal as described in this application. Function (2) represents the equation $P_{out} = K*P_{in}$ (where K is a constant) that is mathematically equivalent to a fixed-constant (linear) frequency multiplication of the input signal. Therefore, the microcontroller unit 88 allows switchability between non-linear and linear multiplication, and the multiplied signal can be outputted to the vitreous cutter at both non-linear and linear cut-rates. Function (3) represents the equation $P_{out} = C*P_{in}^2 + D*P_{in} + E$ (where C, D and E are constants) that provides a somewhat improved non-linear frequency multiplication algorithm, with a higher effective multiplication factor in the mid-range of input cut rates, which gives the surgeon a better control of the cutter 22.

Thus, the digital microcontroller unit 88 allows further refinement and control of the outputted cut rate signal. For example, below a certain threshold input rate (i.e., for input pulse periods longer than a certain threshold limit) a single output pulse can be generated by the processor 84 in response to each input pulse. In other words, the multiplication factor used by the processor 84 is one. Alternatively, above the threshold input rate (for input periods shorter than the threshold limit) either the equation from line (3) or line (4) of Fig. 8 may be applied. The benefit of this approach is the ability to smoothly control the cutter 22 down to very low cut rates, as well as up to very high cut rates.

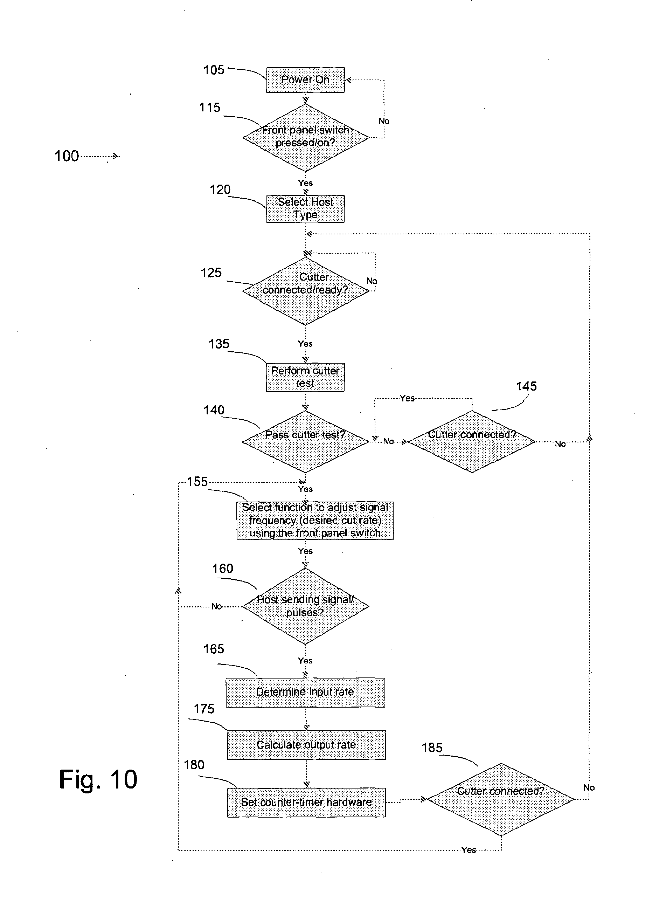

Fig. 10 illustrates a method 100 for operating the non-linear cut-rate multiplier 18 to control the cut-rate of the vitreous cutting system 10. The first step in the method is to turn on the power of the non-linear cut-rate multiplier 18 (step 105). The next step in the method 100 is to verify whether the front panel button 72 of the non-linear cut-rate multiplier 18 is "on" (step 115). If the panel button is not "on", the method goes back to step 105. If the panel button is "on," a user can select the type of "host" or drive unit 14 that is connected to the non-linear cut-rate multiplier 18 (step 120). The display 74 shows the available host types and the corresponding cut rate ranges and cut rate control form (footpedal, panel, or other) for each host type.

In the next step, the method 100 verifies whether the cutter 22 is connected to the multiplier 18 and ready for operation (step 125). If the cutter 22 is ready for operation, the processor 84 performs the cutter test (step 135). If the cutter 22 fails the cutter test (step 140), the processor 84 checks whether the cutter 22 is connected to the multiplier 18 and the drive unit 14 (step 145). The function of the multiplier 18, generally, is to allow the cutter 22 to be driven at a rate much higher than the frequency rate inputted from the drive unit 14, where the drive unit 14 is still controlling the non-linear cut-rate multiplier 18. When the cutter 22 fails the cutter tests and is still connected to the multiplier 18, the method loops in step 145 until the user disconnects the cutter 22 from the multiplier 18. When the cutter 22 is disconnected the method goes back to step 125 where a user connects a new cutter to the multiplier 18.

When the cutter 22 passes the cutter test (step 140), a user can select and change the desired cut rate by using the front panel button (step 155). The processor 84 then determines whether the drive unit 14 is outputting pressure pulses (signal) to the non-linear multiplier 18 (i.e. to check if it is "on") (step 160). If the drive unit is not "on", the method goes back to step 155. If the drive unit is "on", the processor 84 determines the input frequency rate transmitted from the drive unit 14 (step 165). In the next step, the processor 84 calculates the output frequency rate using multiplication based on the desired input of the user (step 175). In step 180, the processor 84 of the MCU 88 sets the output signal of the output counter-timer hardware 92 that is used to drive the high-speed vitreous cutter system 10. Finally, the vitreous cutting system 10 will continue working as long as the cutter 22 is connected to the multiplier 18 and to a power source. When the cutter 22 is connected (step 185), the user can adjust the frequency of the cut-rate at any time by going back to step 155.

Thus, the invention provides, among other things, an accessory in the form of a non-linear cut-rate multiplier that is operable to receive a signal from a drive unit, process the signal, and output an actuation signal to drive a high-speed vitreous cutter at a frequency or rate that is a non-linear multiple of the frequency output by the drive unit to, among other things, provide the user with a plurality of previously unachievable cut-rates.

**Claims**

1. A cut-rate controller for a vitreous cutter, comprising:

   an input for receiving an initial cut-rate signal from a host drive unit;
   a variable non-linear cut-rate multiplying component for multiplying the initial cut-rate signal at a variable non-linear cut-rate;
   an output for providing the multiplied signal at the variable non-linear cut-rate to the vitreous cutter; and
   a linear cut-rate component for multiplying the initial cut-rate signal at a linear cut-rate,
   wherein the cut-rate controller is switchable between the variable non-linear cut-rate and
   the linear cut-rate, and
   wherein the output may provide the multiplied signal at both the variable non-linear cut-rate and the linear cut-rate to the vitreous cutter.

2. A cut-rate controller as claimed in claim 1, wherein the variable non-linear cut-rate multiplying component is comprised of analog components.

3. A cut-rate controller as claimed in claim 1, wherein the variable non-linear cut-rate multiplying component is comprised of digital components.

4. A cut-rate controller as claimed in claim 1, further comprising operator cut-rate controls to switch between the variable non-linear cut-rate and the linear cut-rate.

5. A cut-rate controller as claimed in claim 1, further comprising operator host drive unit controls to select a host type associated with the host drive unit.

6. A cut-rate controller as claimed in claim 1, wherein the cut-rate controller multiplies the initial cut-rate signal at the linear cut-rate for substantially lower cut-rates of the initial cut-rate signal, and multiplies the initial cut-rate signal at the variable non-linear cut-rate for substantially higher cut-rates of the initial cut-rate signal.

7. A method of operating a cut-rate controller for a vitreous cutter, comprising:

   receiving an initial cut-rate signal from a host drive unit;
   multiplying the initial cut-rate signal at a variable non-linear cut-rate;
   outputting the multiplied signal at the variable non-linear cut-rate to the vitreous cutter;
   multiplying the initial cut-rate signal at a linear cut-rate; and
   providing the cut-rate controller switchability between the variable non-linear cut-rate and the linear cut-rate,
   wherein the multiplied signal may be outputted at both the variable non-linear cut-rate and the linear cut-rate to the vitreous cutter.

8. A method of operating a cut-rate controller as claimed in claim 7, wherein the multiplying of the initial cut-rate signal at the variable non-linear cut-rate is performed using analog components.

9. A method of operating a cut-rate controller as claimed in claim 7, wherein the multiplying of the initial cut-rate signal at the variable non-linear cut-rate is performed using digital components.

10. A method of operating a cut-rate controller as claimed in claim 7, further comprising providing operator cut-rate controls to switch between the variable non-linear cut-rate and the linear cut-rate.

11. A method of operating a cut-rate controller as claimed in claim 7, further comprising providing operator host drive unit controls to select a host type associated with the host drive unit.

**12.** A method of operating a cut-rate controller as claimed in claim 7, wherein the cut-rate controller multiplies the initial cut-rate signal at the linear cut-rate for substantially lower cut-rates of the initial cut-rate signal, and multiplies the initial cut-rate signal at the variable non-linear cut-rate for substantially higher cut-rates of the initial cut-rate signal.

**13.** A method of operating a cut-rate controller as claimed in claim 7, wherein the cut-rate controller multiplies the initial cut-rate signal at the linear cut-rate for substantially lower cut-rates of the initial cut-rate signal, multiplies the initial cut-rate signal at a relatively high variable non-linear cut-rate for substantially mid-range cut-rates of the initial cut-rate signal, and multiplies the initial cut-rate signal at a relatively average variable non-linear cut-rate for substantially high cut-rates of the initial cut-rate signal.

**14.** A system for controlling a vitreous cutter, comprising:

a host drive unit for providing an initial cut-rate signal;
a cut-rate controller including an input for receiving the initial cut-rate signal, a variable non-linear cut-rate multiplying component for multiplying the initial cut-rate signal at a variable non-linear cut-rate, an output for providing the multiplied signal at the variable non-linear cut-rate to the vitreous cutter, and a linear cut-rate multiplying component for multiplying the initial cut-rate signal at a linear cut-rate,
wherein the output may provide the multiplied signal at both the variable non-linear cut-rate and the linear cut-rate to the vitreous cutter; and
a vitreous cutter for receiving the multiplied signal and operating at a cut-rate associated with the multiplied signal.

**15.** A system for controlling a vitreous cutter as claimed in claim 14, wherein the cut-rate controller is comprised of analog components.

**16.** A system for controlling a vitreous cutter as claimed in claim 14, wherein the cut-rate controller is comprised of digital components.

**17.** A system for controlling a vitreous cutter as claimed in claim 14, wherein the cut-rate controller further includes operator cut-rate controls to switch between the variable non-linear cut-rate and the linear cut-rate.

**18.** A system for controlling a vitreous cutter as claimed in claim 14, wherein the cut-rate controller further includes operator host drive unit controls to select a host type associated with the host drive unit.

**19.** A system for controlling a vitreous cutter as claimed in claim 14, wherein the cut-rate controller multiplies the initial cut-rate signal at the linear cut-rate for substantially lower cut-rates of the initial cut-rate signal and multiplies the initial cut-rate signal at the variable non-linear cut-rate for substantially higher cut-rates of the initial cut-rate signal.

**Patentansprüche**

**1.** Schneidraten-Steuereinheit für einen Glaskörperschrieider, die umfasst:

einen Eingang zum Empfangen eines anfänglichen Schneidratensignals von einer Hauptantriebseinheit;
eine Komponente zum Multiplizieren mit einer nichtlinearen Schneidrate, um das anfängliche Schneidratensignal mit einer variablen, nichtlinearen Schneidrate zu multiplizieren;
einen Ausgang, um das mit der variablen, nichtlinearen Schneidrate multiplizierte Signal für den Glaskörperschneider bereitzustellen; und
eine Komponente für eine lineare Schneidrate, um das anfängliche Schneidratensignal mit einer linearen Schneidrate zu multiplizieren,
wobei die Schneidraten-Steuereinheit zwischen der variablen, nichtlinearen Schneidrate und der linearen Schneidrate umschaltbar ist und
wobei der Ausgang sowohl das mit der variablen, nichtlinearen Schneidrate multiplizierte Signal als auch das mit der linearen Schneidrate multiplizierte Signal für den Glaskörperschneider bereitstellen kann.

**2.** Schneidraten-Steuereinheit nach Anspruch 1, wobei die Komponente zum Multiplizieren mit einer variablen, nichtlinearen Schneidrate analoge Komponenten umfasst.

**3.** Schneidraten-Steuereinheit nach Anspruch 1, wobei die Komponente zum Multiplizieren mit einer variablen, nichtlinearen Schneidrate digitale Komponenten umfasst.

4. Schneidraten-Steuereinheit nach Anspruch 1, die ferner Bedienerschneidraten-Steuerungen umfasst, um zwischen der variablen, nichtlinearen Schneidrate und der linearen Schneidrate umzuschalten.

5. Schneidraten-Steuereinheit nach Anspruch 1, die ferner Bedienerhauptantriebseinheit-Steuerungen umfasst, um einen der Hauptantriebseinheit zugeordneten Haupttyp auszuwählen.

6. Schneidraten-Steuereinheit nach Anspruch 1, wobei die Schneidraten-Steuereinheit das anfängliche Schneidratensignal mit der linearen Schneidrate für im Wesentlichen niedrigere Schneidraten des anfänglichen Schneidratensignals multipliziert und das anfängliche Schneidratensignal mit der variablen, nichtlinearen Schneidrate für im Wesentlichen höhere Schneidraten des anfänglichen Schneidratensignals multipliziert.

7. Verfahren zum Betreiben einer Schneidraten-Steuereinheit für einen Glaskörperschneider, das umfasst:

Empfangen eines anfänglichen Schneidratensignals von einer Hauptantriebseinheit;
Multiplizierten des anfänglichen Schneidratensignals mit einer variablen, nichtlinearen Schneidrate;
Ausgeben des mit der variablen, nichtlinearen Schneidrate multiplizierten Signals an den Glaskörperschneider;
Multiplizieren des anfänglichen Schneidratensignals mit einer linearen Schneidrate; und
Vorsehen einer Umschaltbarkeit der Schneidraten-Steuereinheit zwischen der variablen, nichtlinearen Schneidrate und der linearen Schneidrate,
wobei sowohl das mit der variablen, nichtlinearen Schneidrate multiplizierte Signal als auch das mit der linearen Schneidrate multiplizierte Signal an den Glaskörperschneider ausgegeben werden kann.

8. Verfahren zum Betreiben einer Schneidraten-Steuereinheit nach Anspruch 7, wobei das Multiplizieren des anfänglichen Schneidratensignals mit der variablen, nichtlinearen Schneidrate unter Verwendung analoger Komponenten ausgeführt wird.

9. Verfahren zum Betreiben einer Schneidraten-Steuereinheit nach Anspruch 7, wobei das Multiplizieren des anfänglichen Schneidratensignals mit der variablen, nichtlinearer Schneidrate unter Verwendung digitaler Komponenten ausgeführt wird.

10. Verfahren zum Betreiben einer Schneidraten-Steuereinheit nach Anspruch 7, das ferner das Vorsehen von Bedienerschneidraten-Steuerungen umfasst, um zwischen der variablen, nichtlinearen Schneidrate und der linearen Schneidrate umzuschalten.

11. Verfahren zum Betreiben einer Schneidraten-Steuereinheit nach Anspruch 7, das ferner das Vorsehen von Bedienerhauptantriebseinheit-Steuerungen umfasst, um einen der Hauptantriebseinheit zugeordneten Haupttyp auszuwählen.

12. Verfahren zum Betreiben einer Schneidraten-Steuereinheit nach Anspruch 7, wobei die Schneidraten-Steuereinheit das anfängliche Schneidratensignal mit der linearen Schneidrate für im Wesentlichen niedrigere Schneidraten des anfänglichen Schneidratensignals multipliziert und das anfängliche Schneidratensignal mit der variablen, nichtlinearen Schneidrate für im Wesentlichen höhere Schneidraten des anfänglichen Schneidratensignals multipliziert.

13. Verfahren zum Betreiben einer Schneidraten-Steuereinheit nach Anspruch 7, wobei die Schneidraten-Steuereinheit das anfängliche Schneidratensignal mit der linearen Schneidrate für im Wesentlichen niedrigere Schneidraten des anfänglichen Schneidratensignals multipliziert, das anfängliche Schneidratensignal mit einer verhältnismäßig hohen variablen, nichtlinearen Schneidrate für im Wesentlichen mittlere Schneidraten des anfänglichen Schneidratensignals multipliziert und das anfängliche Schneidratensignal mit einer verhältnismäßig durchschnittlichen variablen, nichtlinearen Schneidrate für im Wesentlichen hohe Schneidraten des anfänglichen Schneidratensignals multipliziert.

14. System zum Steuern eines Glaskörperschneiders, das umfasst:

eine Hauptantriebseinheit, um ein anfängliches Schneidratensignal bereitzustellen;
eine Schneidraten-Steuereinheit, die einen Eingang zum Empfangen des anfänglichen Schneidratensignals, eine Komponente zum Multiplizieren mit einer variablen, nichtlinearen Schneidrate, um das anfängliche Schneidratensignal mit einer variablen, nichtlinearen Schneidrate zu multiplizieren, einen Ausgang zum Bereitstellen des mit der variablen, nichtlinearen Schneidrate multiplizierten Signals für den Glaskörperschneider und eine Komponente zum Multiplizieren mit einer linearen Schneidrate zum Multiplizieren des anfänglichen Schneidratensignals mit einer nichtlinearen Schneidrate enthält,

wobei der Ausgang sowohl das mit der variablen, nichtlinearen Schneidrate multiplizierte Signal als auch das mit der linearen Schneidrate multiplizierte Signal für den Glaskörperschneider bereitstellen kann; und einen Glaskörperschneider zum Empfangen des multiplizierten Signals und zum Arbeiten mit einer Schneidrate, die dem multiplizierten Signal zugeordnet ist.

**15.** System zum Steuern eines Glaskörperschneiders nach Anspruch 14, wobei die Schneidraten-Steuereinheit analoge Komponenten umfasst.

**16.** System zum Steuern eines Glaskörperschneiders nach Anspruch 14, wobei die Schneidraten-Steuereinheit digitale Komponenten umfasst.

**17.** System zum Steuern eines Glaskörperschneiders nach Anspruch 14, wobei die Schneidraten-Steuereinheit ferner Bedienerschneidraten-Steuerungen umfasst, um zwischen der variablen, nichtlinearen Schneidrate und der linearen Schneidrate umzuschalten.

**18.** System zum Steuern eines Glaskörperschneiders nach Anspruch 14, wobei die Schneidraten-Steuereinheit Bedienerhauptantriebseinheit-Steuerungen umfasst, um einen der Hauptantriebseinheit zugeordneten Haupttyp auszuwählen.

**19.** System zum Steuern eines Glaskörperschneiders nach Anspruch 14, wobei die Schneidraten-Steuereinheit das anfängliche Schneidratensignal mit der linearen Schneidrate für im Wesentlichen niedrigere Schneidraten des anfänglichen Schneidratensignals multipliziert und das anfängliche Schneidratensignal mit der variablen, nichtlinearen Schneidrate für im Wesentlichen höhere Schneidraten des anfänglichen Schneidratensignals multipliziert.

**Revendications**

**1.** Régulateur de vitesse de coupe pour dispositif de coupe vitreux, comprenant :

une entrée permettant de recevoir un signal de vitesse de coupe initiale provenant d'une unité d'actionnement hôte ;
un composant multiplicateur de la vitesse de coupe non linéaire variable permettant de multiplier le signal de vitesse de coupe initiale à une vitesse de coupe non linéaire variable ;
une sortie permettant de transmettre le signal multiplié à la vitesse de coupe non linéaire variable au dispositif de coupe vitreux ; et
un composant de vitesse de coupe linéaire permettant de multiplier le signal de vitesse de coupe initiale à une vitesse de coupe linéaire, dans lequel le régulateur de vitesse de coupe peut commuter entre la vitesse de coupe non linéaire variable et la vitesse de coupe linéaire, et
dans lequel la sortie peut transmettre au dispositif de coupe vitreux le signal multiplié à la fois à la vitesse de coupe non linéaire variable et à la vitesse de coupe linéaire.

**2.** Régulateur de vitesse de coupe selon la revendication 1, dans lequel le composant multiplicateur de la vitesse de coupe non linéaire variable est constitué de composants analogiques.

**3.** Régulateur de vitesse de coupe selon la revendication 1, dans lequel le composant multiplicateur de la vitesse de coupe non linéaire variable est constitué de composants numériques.

**4.** Régulateur de vitesse de coupe selon la revendication 1, comprenant en outre des commandes d'opérateur de la vitesse de coupe permettant de commuter entre la vitesse de coupe non linéaire variable et la vitesse de coupe linéaire.

**5.** Régulateur de vitesse de coupe selon la revendication 1, comprenant en outre des commandes d'opérateur de l'unité d'actionnement hôte permettant de sélectionner un type hôte associé à l'unité d'actionnement hôte.

**6.** Régulateur de vitesse de coupe selon la revendication 1, dans lequel le régulateur de vitesse de coupe multiplie le signal de vitesse de coupe initiale à la vitesse de coupe linéaire pour obtenir des vitesses de coupe sensiblement inférieures du signal de vitesse de coupe initiale, et multiplie le signal de vitesse de coupe initiale à la vitesse de coupe non linéaire variable pour obtenir des vitesses de coupe sensiblement supérieures du signal de vitesse de coupe initiale.

**7.** Procédé de mise en oeuvre d'un régulateur de vitesse de coupe pour un dispositif de coupe vitreux, comprenant :

la réception d'un signal de vitesse de coupe initiale provenant d'une unité d'actionnement hôte ;
la multiplication du signal de vitesse de coupe initiale à une vitesse de coupe non linéaire variable ;
l'envoi au dispositif de coupe vitreux du signal multiplié à la vitesse de coupe non linéaire variable ;
la multiplication du signal de vitesse de coupe initiale à une vitesse de coupe linéaire ; et
la fourniture au régulateur de vitesse de coupe de la possibilité de commuter entre la vitesse de coupe non linéaire variable et la vitesse de coupe linéaire,
dans lequel le signal multiplié peut être envoyé au dispositif de coupe vitreux à la fois à la vitesse de coupe non linéaire variable et à la vitesse de coupe linéaire.

**8.** Procédé de mise en oeuvre d'un régulateur de vitesse de coupe selon la revendication 7, dans lequel la multiplication du signal de vitesse de coupe initiale à la vitesse de coupe non linéaire variable est effectuée à l'aide de composants analogiques.

**9.** Procédé de mise en oeuvre d'un régulateur de vitesse de coupe selon la revendication 7, dans lequel la multiplication du signal de vitesse de coupe initiale à la vitesse de coupe non linéaire variable est effectuée à l'aide de composants numériques.

**10.** Procédé de mise en oeuvre d'un régulateur de vitesse de coupe selon la revendication 7 comprenant en outre la fourniture de commandes d'opérateur de la vitesse de coupe permettant de commuter entre la vitesse de coupe non linéaire variable et la vitesse de coupe linéaire.

**11.** Procédé de mise en oeuvre d'un régulateur de vitesse de coupe selon la revendication 7, comprenant en outre la fourniture de commandes d'opérateur de l'unité d'actionnement hôte permettant de sélectionner un type hôte associé à l'unité d'actionnement hôte.

**12.** Procédé de mise en oeuvre d'un régulateur de vitesse de coupe selon la revendication 7, dans lequel le régulateur de vitesse de coupe multiplie le signal de vitesse de coupe initiale à la vitesse de coupe linéaire pour obtenir des vitesses de coupe sensiblement inférieures du signal de vitesse de coupe initiale, et multiplie le signal de vitesse de coupe initiale à la vitesse de coupe non linéaire variable pour obtenir des vitesses de coupe sensiblement supérieures du signal de vitesse de coupe initiale.

**13.** Procédé de mise en oeuvre d'un régulateur de vitesse de coupe selon la revendication 7, dans lequel le régulateur de vitesse de coupe multiplie le signal de vitesse de coupe initiale à la vitesse de coupe linéaire pour obtenir des vitesses de coupe sensiblement inférieures du signal de vitesse de coupe initiale, multiplie le signal de vitesse de coupe initiale à une vitesse de coupe non linéaire variable relativement élevée pour obtenir des vitesses de coupe de sensiblement milieu de gamme du signal de vitesse de coupe initiale, et multiplie le signal de vitesse de coupe initiale à une vitesse de coupe non linéaire variable sensiblement moyenne pour obtenir des vitesse de coupe sensiblement élevées du signal de vitesse de coupe initiale.

**14.** Système de commande d'un dispositif de coupe vitreux, comprenant :

une unité d'actionnement hôte permettant de fournir un signal de vitesse de coupe initiale ;
un régulateur de vitesse de coupe comprenant une entrée permettant de recevoir le signal de vitesse de coupe initiale, un composant multiplicateur de la vitesse de coupe non linéaire variable permettant de multiplier le signal de vitesse de coupe initiale à une vitesse de coupe non linéaire variable, une sortie permettant de transmettre au dispositif de coupe vitreux le signal multiplié à la vitesse de coupe non linéaire variable, et un composant multiplicateur de la vitesse de coupe linéaire permettant de multiplier le signal de vitesse de coupe initiale à une vitesse de coupe linéaire,
dans lequel la sortie peut transmettre au dispositif de coupe vitreux le signal multiplié à la fois à la vitesse de coupe non linéaire variable et à la vitesse de coupe linéaire ; et

un dispositif de coupe vitreux permettant de recevoir le signal multiplié et de fonctionner à une vitesse de coupe associée au signal multiplié.

15. Système de commande d'un dispositif de coupe vitreux selon la revendication 14, dans lequel le régulateur de vitesse de coupe est constitué de composants analogiques.

16. Système de commande d'un dispositif de coupe vitreux selon la revendication 14, dans lequel le régulateur de vitesse de coupe est constitué de composants numériques.

17. Système de commande d'un dispositif de coupe vitreux selon la revendication 14, dans lequel le régulateur de vitesse de coupe comprend en outre des commandes d'opérateur de la vitesse de coupe permettant de commuter entre la vitesse de coupe non linéaire variable et la vitesse de coupe linéaire.

18. Système de commande d'un dispositif de coupe vitreux selon la revendication 14, dans lequel le régulateur de vitesse de coupe comprend en outre des commandes d'opérateur de l'unité d'actionnement hôte permettant de sélectionner un type hôte associé à l'unité d'actionnement hôte.

19. Système de commande d'un dispositif de coupe vitreux selon la revendication 14, dans lequel le régulateur de vitesse de coupe multiplie le signal de vitesse de coupe initiale à la vitesse de coupe linéaire pour obtenir des vitesses de coupe sensiblement inférieures du signal de vitesse de coupe initiale et multiplie le signal de vitesse de coupe initiale à la vitesse de coupe non linéaire variable pour obtenir des vitesses de coupe sensiblement supérieures du signal de vitesse de coupe initiale.

FIG. 1

EP 2 434 997 B1

EP 2 434 997 B1

FIG. 2

16

FIG. 3

FIG. 4

FIG. 5

FIG. 6

| Selection Letter | Cut Rate Range | Cut Rate Control |
|:---:|:---:|:---:|
| A | 2500 CPM Max | Footpedal |
| B | 2000 CPM Max | Footpedal |
| C | 1500 CPM Max | Footpedal |
| D | 800 CPM Max | Footpedal |
| E | 750 CPM Max | Footpedal |
| F | 2500 CPM Max | Panel |
| G | 1500 CPM Max | Panel |
| H | 800 CPM Max | Panel |

# Fig. 7

| Host Type | Maximum Rate or Multiplier |
|:---:|:---:|
| A | 8000, 6000, 4000, 3000 |
| B | 8000, 6000, 4000, 3000 |
| C | 8000, 6000, 4000, 3000 |
| D | 8000, 6000, 4000, 3000 |
| E | 8000, 6000, 4000, 3000 |
| F | 4x, 3x, 2x, 1x |
| G | 5x, 4x, 3x, 1x |
| H | 10x, 5x, 3x, 2x |

# Fig. 8

FIG. 9

100 ·········➤

**Fig. 10**

105 — Power On

115 — Front panel switch pressed/on?
No

Yes

120 — Select Host Type

125 — Cutter connected/ready?
No

Yes

135 — Perform cutter test

140 — Pass cutter test?
Yes
145 — Cutter connected?
No
No

Yes

155 — Select function to adjust signal frequency (desired cut rate) using the front panel switch

Yes

160 — Host sending signal/pulses?
No

Yes

165 — Determine input rate

175 — Calculate output rate

180 — Set counter-timer hardware

185 — Cutter connected?
No

Yes

FIG. 11

**EP 2 434 997 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3884238 A
- US 3815604 A
- US 6575990 B, Wang